Europäisches Patentamt

European Patent Office

Office européen des brevets

(11) Publication number: 0 219 053 A2

# (12) EUROPEAN PATENT APPLICATION

(21) Application number: 86113898.0

(22) Date of filing: 07.10.86

(51) Int. Cl.⁴: A61M 1/34

(30) Priority: 07.10.85 US 785135

(43) Date of publication of application:
22.04.87 Bulletin 87/17

(84) Designated Contracting States:
DE FR GB IT

(71) Applicant: AMERICAN HOSPITAL SUPPLY
CORPORATION
One Baxter Parkway
Deerfield Illinois 60015(US)

(72) Inventor: Hsu, Li-Chien
26572 Avenida DeSeo
Mission Viejo California 92691(US)
Inventor: Balding, David P.
26552 Tampico Place
Mission Viejo California 92691(US)
Inventor: Clarke, Rolf W.
304 Collins Ave
Balboa Island California 92602(US)
Inventor: Mosch, Karl E.
63 E. Yale Loop
Irvine California 92714(US)

(74) Representative: Patentanwälte Grünecker,
Kinkeldey, Stockmair & Partner
Maximilianstrasse 58
D-8000 München 22(DE)

(54) Blood filtration devices with heparin coated filter elements.

(57) Heparin coated blood filtration devices prepared in a one-step process by applying a heparin coating to the porous substrate of a blood filter element. The heparin coating provides improved porous blood filtration devices useful, for example, in cardiopulmonary bypass arterial blood filters.

FIG. 5

## BLOOD FILTRATION DEVICES WITH HEPARIN COATED FILTER ELEMENTS

### Field of the Invention

The invention relates to improved blood filtration devices.

### Background of the Invention

It is frequently desirable in medical practice, for example during surgery, to cause blood to flow through an extracorporeal circuit. When this is done, undesirable foreign matter such as tissue or bone debris, gaseous emboli, clots, or other matter may come into contact with the blood which it is desirable to filter from the blood before the blood returns to the body. In other medical procedures, desired blood filtration may be accomplished through use of an intracorporeal filter.

Examples of blood filtration devices are known blood filter assembly structures. Among these are the blood filters disclosed in Gordon, U.S. application 124,312, filed February 25, 1980; Esmond, U.S. Patent 3,795,088; Luceyk et al., U.S. Patent 4,038,194; and Mouwen, U.S. Patent 4,056,476, the disclosures of which are incorporated herein by reference.

The present invention provides blood filtration devices such as arterial blood filters which realize a variety of improvements by applying a heparin-cationic surfactant coating to the porous substrate of the blood filter element used in the blood filtration device. First, the invention achieves improved compatibility between the filter element and blood and thus reduces the possibility of blood coagulation, protein adsorption, or platelet retention within the filter element. These phenomena may, over the extended use of a blood filter, lead to partial occlusion of filter pores and a resulting diminution of filtering capacity. By delaying accumulation within the filter of such blood materials, the present invention allows maximum filtration capacity to be maintained for a longer time.

A second improvement relates to the ease of priming blood filtration devices which utilize the present invention. Because the heparin coated filters described herein exhibit better wettability than uncoated filters, priming before use is greatly facilitated.

A third improvement of the present invention resides in the process of applying a dilute heparin coating solution to a porous filter substrate so as to avoid bridging of the filter pores by the deposited heparin-cationic surfactant complex. Previously known heparin coating methods yielded coats which were unacceptably thick and which would, if applied to the porous substrates of the blood filtration devices described herein, occlude the minute pores of such substrates.

Other and additional improvements will be apparent upon a reading of the entire specification.

Various heparin coating compounds have been applied in the past to non-filter devices. For example, heparin coated catheters are known, and are available commercially as U.S.C.I. Thermodilution Catheters with Safe-T-Coat heparin coating, or American Edwards Multi-Med Catheters with Thromboshield. Heparin coated tubing is described by H. Roohk, et al., in Trans. Am. Soc. Artif. Intern. Organs, vol. 23, pages 152-61 (1977). In addition, Higley, U.S. Patent No. 4,308,145, discloses a polycarbonate hemodialysis membrane with the suggestion that its surface may be heparin coated. Such devices do not, however, encounter the same problems as the porous blood filtration devices of the present invention.

For example, the physical filtration of blood through a porous substrate is not a relevant consideration in these prior contexts. Blood does not flow through the coated wall of a catheter, a tube, or a dialysis membrane; rather, the blood merely contacts a surface which is intended precisely to prevent flow-through. The dialysis membrane of Patent No. 4,308,145, for example, suggests a heparin coated leak-proof surface which seals off blood from a dialysate solution but allows selective diffusion of toxin solutes. Heparin applied to such a surface does not realize the unique advantages of heparin applied to a network of microscopic pores, such as improved flow-through filtration capacity and improved priming properties. Thus, the present invention teaches specific improvements in blood filtration devices which are not contemplated by prior heparin coating art.

Moreover, processes described in the prior art for applying heparin coatings to surfaces are not acceptable in the context of blood filters. For example, the method disclosed in Masuhara, U.S. Patent No. 3,717,502, involves use of a coating solvent capable of dissolving the surface to be coated. Such solvents would be destructive of the pore matrix of the filters used in the present invention, by, for example, "locking up" the fibers of a woven filter screen. Similarly, the coating method described by K. Amplatz in Investigative Radiology, Vol. 6, pages 280-89 (1971), yields dip-coats which would be unacceptable for common blood filters in that the filter pores would be bridged and occluded by the deposited heparin coat.

## SUMMARY OF THE INVENTION

The present invention provides improved porous blood filtration devices, such as improved cardiopulmonary bypass arterial blood filters, having heparin coated porous substrates as blood filter elements. The heparin coating on the porous substrates of the present invention enhances the blood compatibility of the blood filtration devices and reduces undesirable adhesion of blood components. The application of a heparin coating to the porous filter substrate of a blood filtration device is accomplished by the use of heparin-cationic surfactant complexes, as described in the detailed description of the invention.

## BRIEF DESCRIPTION OF THE DRAWINGS

FIGURE I is a graph which illustrates, as a function of time, the pre-filter fluid pressure of blood flowing through heparin coated arterial blood filters of the present invention and through an uncoated control filter. The increase in pressure associated with the uncoated control reflects the gradual accumulation of solid blood materials within the filter pores, as compared to near uniform pressure measured for heparin coated filters.

FIGURE 2 is a graph which illustrates reduced platelet retention associated with heparin coated arterial blood filters of the present invention, as indicated by relatively higher platelet counts in filtered blood.

FIGURE 3 is a graph which illustrates the reduced rates of fibrinogen adsorption of the heparin coated filters of the present invention compared to uncoated filters. Fibrinogen adsorption is believed to be a useful index of the compatibility of a biomaterial with blood.

FIGURE 4 is a tabulation of data which demonstrates improved priming characteristics of heparin coated arterial blood filters of the present invention compared to uncoated arterial filters. These data reflect residual gas remaining in the filters following retrograde filling with isotonic saline.

FIGURE 5 is a side view of an arterial blood filtration device of the present invention.

FIGURE 6 is a partial cross-sectional side view of the arterial blood filtration device of Figure 5 taken along line 6-6 of FIG. 5.

FIGURE 7 is a cross-sectional view of a heparin coated pleated blood filter element for the arterial blood filtration device of Figure 5 taken along line 7-7 of FIG. 6.

FIGURE 8 is a cutaway plan view of a collapsible venous blood reservoir with an integral heparin coated filter screen.

FIGURE 9 is a cutaway side elevation of a blood oxygenator with an integral heparin coated filter screen.

## DETAILED DESCRIPTION OF THE INVENTION

The present invention relates to a novel application of a heparin coating to the porous filter substrate of a blood filter element used in a blood filtration device, such as an arterial blood filter, to delay the formation and accumulation o coagulated blood material or other proteinaceous matter normally associated with the presence of foreign bodies in the bloodstream. The invention provides a transparent coating formed by complex of a cationic surfactant and heparin.

In order to promote a stable bond between the porous filter substrate and the heparin complex, it is preferred to use a cationic surfactant containing at least one alkyl chain of at least two, and preferably at least four, carbon atoms. Surface active agents having a long hydrocarbon chain are preferred, particularly those containing at least one hydrocarbon chain of from about 8 to about 18 carbon atoms. Suitable surfactants can be of a varied nature and include primary, secondary and tertiary amines and their salts, as well as quaternary ammonium compounds, in which at least one alkyl group has a chain length longer than about four carbon atoms and preferably longer than about eight carbon atoms. Examples of preferred quaternary ammonium salts are dimethylalkylbenzyl ammonium chloride compounds, tridodecylmethyl ammonium chloride, and tricaprylmethyl ammonium chloride. A preferred quaternary ammonium salt is benzalkonium chloride, which is a mixture of dimethylalkylbenzyl ammonium chloride compounds wherein each distinct compound in the mixture contains an alkyl group with a distinct chain length of from about 8 to about 18 carbon atoms. Benzalkonium chloride meeting the U.S.P. standard is especially preferred. This standard is found in the U.S. Pharmacopeia, page 1211 (1980). Also preferred is any single dimethylalkylbenzyl ammonium chloride compound wherein the alkyl group is selected singly from those groups having a chain length of from about 8 to about 18 carbon atoms.

Benzalkonium heparin, a complex formed from heparin and benzalkonium chloride, can be prepared, for example, by slowly adding benzalkonium chloride aqueous solution to an aqueous solution of heparin (preferably sodium heparin) until the precipitate ceases to occur. The precipitate is then thoroughly washed with purified water and dried. The complex so prepared is dissolved in alcohols or other organic solvents and then reprecipitated in water. The purification process is repeated until the complex is free from unbound benzalkonium chloride.

The coating process of the present invention may be used to coat porous filter substrates having a wide range of pore sizes. For the purpose of the blood filtration devices of the present invention, substrates having pores of typical size ranging from about 10 microns to about 150 microns are preferred. Different blood filtration devices may advantageously utilize different ranges of pore size. A typical pore size ranging from about 20 microns to about 45 microns is particularly preferred for arterial blood filters, while a pore size ranging from about 80 microns to about 120 microns is particularly preferred for venous flood filtration, as, for example, in a venous blood reservoir.

The pore sizes indicated represent nominal cross-sectional pore dimensions. In practice, pore size may be calculated by reference to the size of material filtered from the blood during the filtration process. In the case of certain filter materials, for example, woven or knitted filter screens, pore size may be calculated by reference to the number and size of substrate filaments in a given region.

Porous materials which may be coated with heparin in accordance with the present invention are characterized by the pore sizes indicated above. These porous materials include, for example, substrates of polyesters, nylon, polyolefin, acrylic, fluorinated hydrocarbons, polyurethane, polycarbonate and cellulose and its derivatives. Suitable polyesters include polyethylene terephthalate and polybutylene terephthalate. Suitable polyolefins include polyethylene and polypropylene as well as copolymers of ethylene and propylene. Polytetrafluoroethylene is an example of a suitable fluorinated hydrocarbon; cellulose acetate is an example of a suitable cellulose derivative.

Porous substrates useful in the practice of the present invention may also be made from resin foams made from thermoplastic or thermosetting resins. Suitable thermoplastic resin foams include polyvinylchloride foam, polyethylene foam, and polystyrene foam. Suitable thermosetting resin foams include phenolic foams, polyurethane foams, and silicone foams.

Porous substrates in the form of woven or knitted screens are also useful in the practice of the present invention. Materials suitable for the construction of such screens include, for example, polyester, nylon, polyurethane and cellulose and its derivatives. Porous felt substrates, which may be constructed of, for example, polyester or polyolefin, are also useful in the practice of the present invention.

Coating solutions are prepared by dissolving appropriate amounts of heparin-cationic surfactant complexes in organic solvents. Typical organic solvents include, for example, lower alcohols and toluene. Typical lower alcohols include from about 2 to about 4 carbon atoms. 2-propanol is a preferred organic solvent. The solution concentration ranges from about 0.02% to about 10% (w/v). The filter elements are dip-coated in these solutions and then dried until all traces of solvent are removed.

The heparin-cationic surfactant complexes may be further secured or immobilized on the pore walls of the filter substrate by intermolecular cross-linking of individual heparin or heparin complex molecules with an aldehyde, such as formaldehyde, or a dialdehyde, such as glutardialdehyde, thereby forming a network between the complex molecules. Thus, filters coated with heparin complex may be immersed in a glutardialdehyde solution of about 0.1 to about 0.5% (w/v) at pH of 2 to 10 at about 25° to about 60°C from about 10 to about 60 minutes to facilitate the cross-linking reaction.

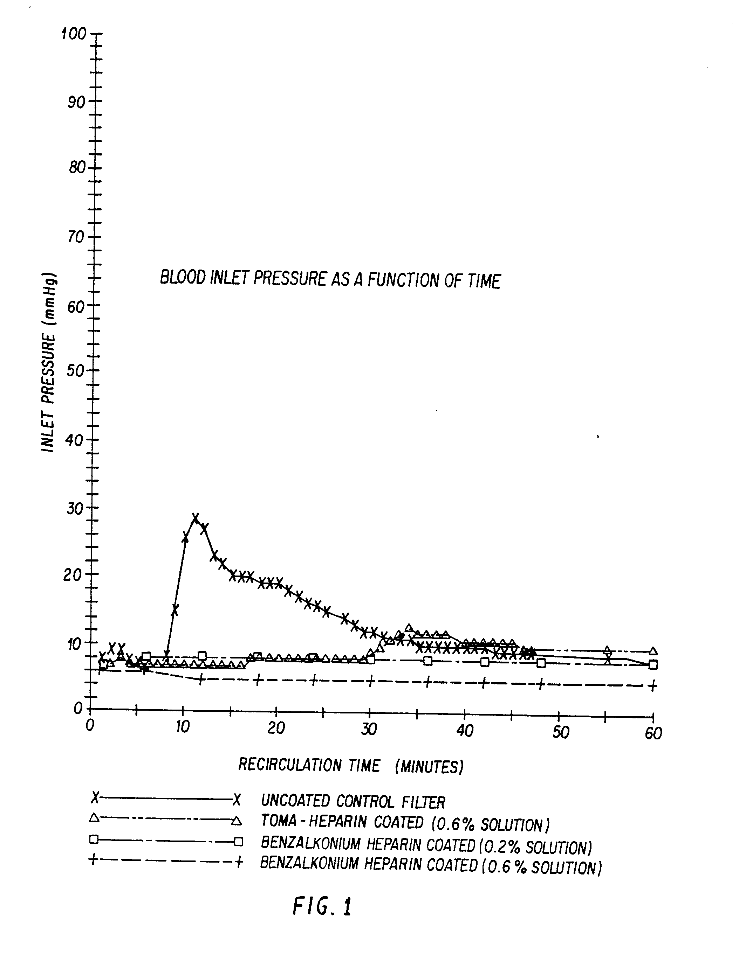

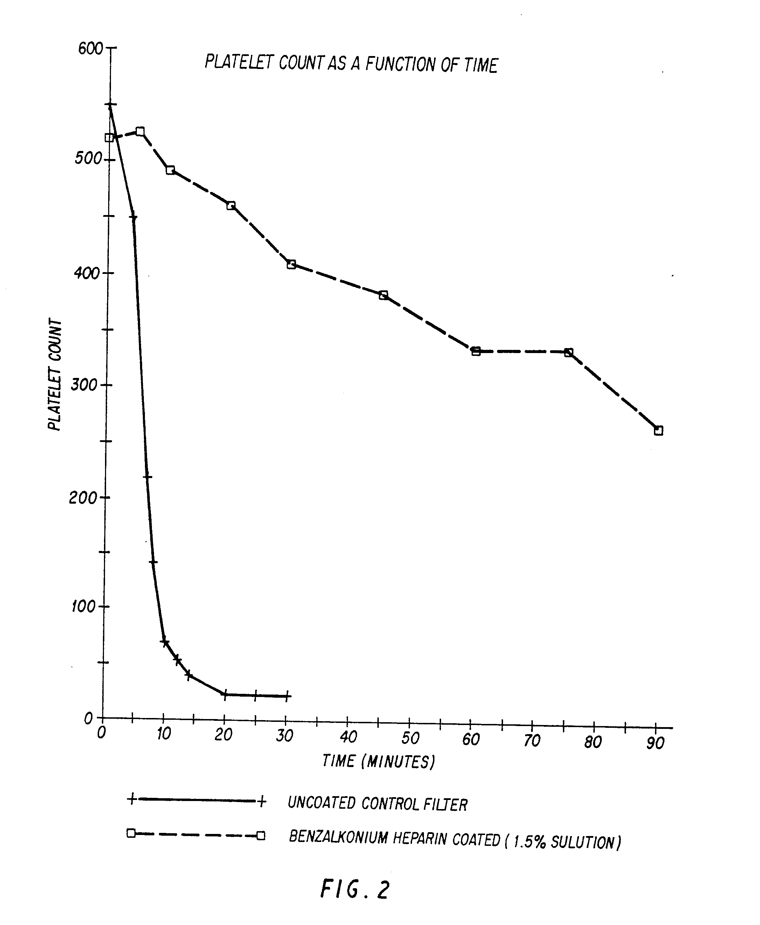

Evaluations of coated filters have shown that arterial blood filtration devices utilizing the heparin coated filter elements of the present invention have improved performance compared to uncoated filters. Improved performance is indicated by reduced fluid pressure buildup associated with coated filter elements compared to uncoated filters when blood is passed through the filter, as illustrated in FIG. 1. Also, improved compatibility with blood is demonstrated by reduced platelet retention, as illustrated in FIG. 2.

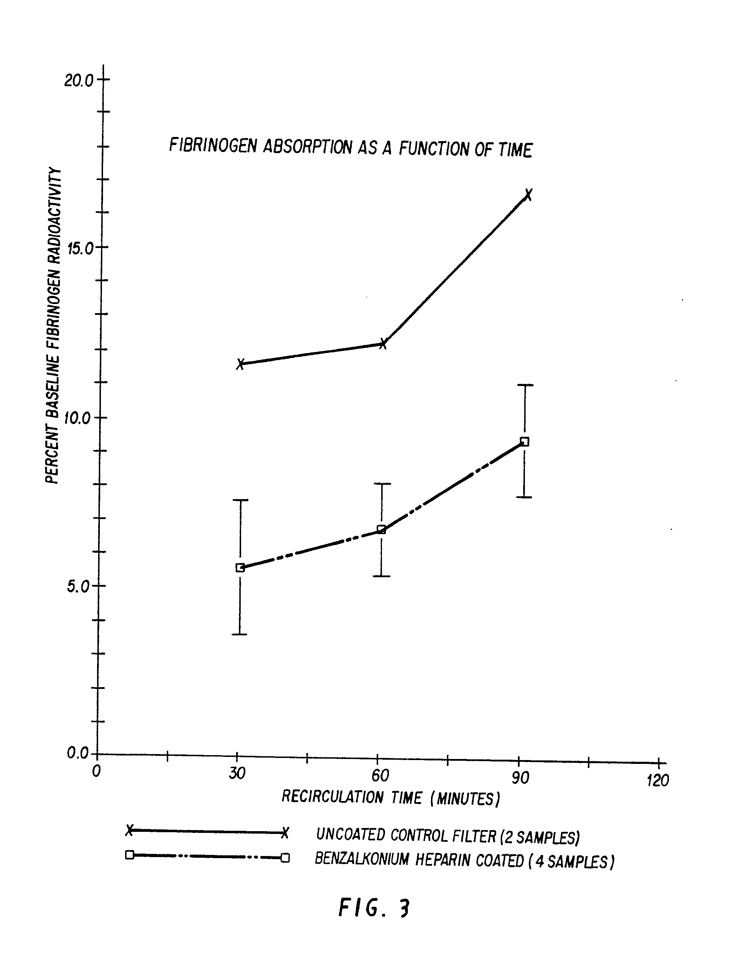

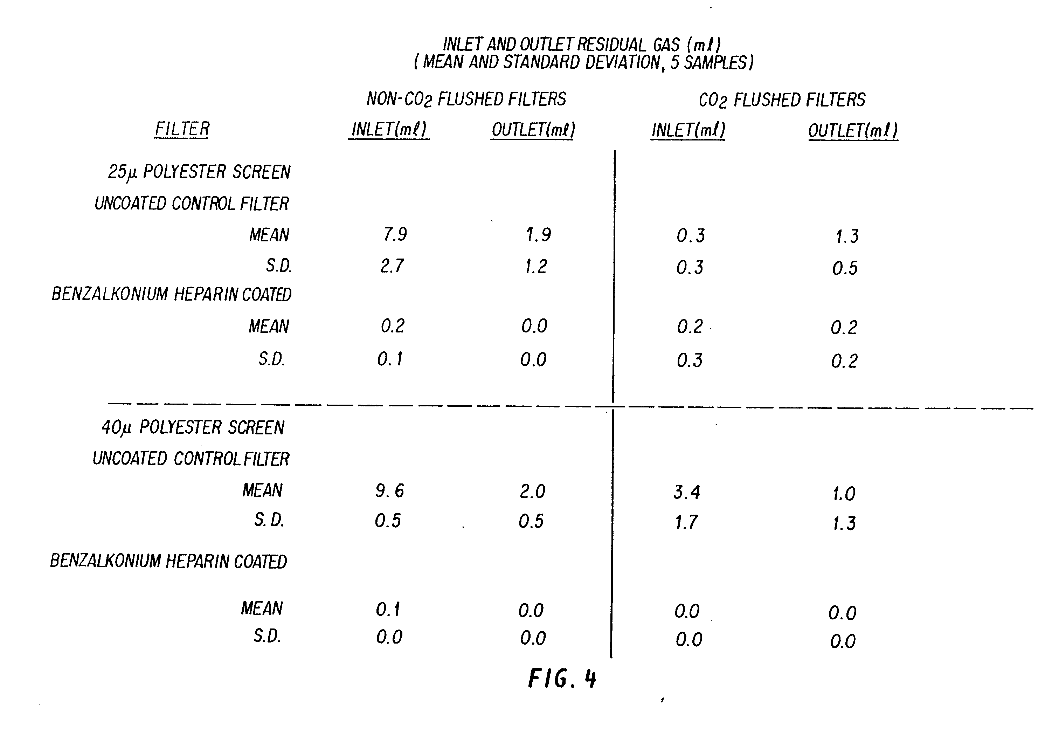

The data presented in FIG. 3 also demonstrate improved blood compatibility, by virtue of reduced fibrinogen adsorption within the coated filter of the present invention. The data in FIG. 4 demonstrate the improved priming characteristics of the coated filters of the present invention as compared to the uncoated filters. This is believed to be a result of the significant improvement in the wettability of the filter element caused by the heparin coating.

As mentioned above, a preferred heparin-cationic surfactant complex used in the present invention is a heparin quaternary ammonium complex. Commercially available heparin quaternary ammonium complexes which may be used in the practice of the present invention are benzalkonium heparin and tridodecyl methyl ammonium heparin - (TDMA-heparin). The benzalkonium moiety contains three alkyl side chains. One of the alkyl side chains comprises a mixture of alkyls which range in length from about 8 to about 18 carbon atoms. The TDMA moiety contains four alkyl side chains, three of which are alkyl groups having about 12

carbon atoms. These long alkyl side chains are believed to undergo weak van der Waals interactions with the porous substrate material, such as the polyester fibers of a filter element. The heparin molecule is in turn held to the porous substrate by virtue of ionic bonds between the negatively charged heparin and the positively charged quaternary ammonium group.

Benzalkonium heparin complex is a white waxy compound which is insoluble in water. Benzalkonium heparin complex is commercially available and can be obtained, for example, from Sterilization Technical Services, 7500 West Henrietta Road, Rush NY 14543. TDMA-heparin is available from Polysciences, Inc., 400 Valley Road, Warrington, PA 18976.

In the practice of a preferred embodiment of the present invention, heparin benzalkonium complex is dissolved in an alcohol such as 2-propanol to produce a solution of concentration between about 0.02 to about 10% (w/v). A blood filtration device such as an arterial blood filter is dipped into this solution, removed, and then dried. A thin, transparent waxy film of benzalkonium heparin complex is thus deposited on the pore walls of the filter substrate, or on the individual fibers of, e.g., a woven polyester filter screen.

The preferred working concentration of a solution of benzalkonium heparin complex in 2-propanol is between about 0.2 to about 1.0% (w/v), most preferably between about 0.2 to about 0.6% - (w/v). This provides a concentration which allows sufficient heparin to remain on the pore walls of the filter substrate, yet not depositing so much compound within the pores that bridging occurs across the pores, or in the interstices between the individual fibers of, e.g., a woven polyester filter screen.

Quaternary ammonium complexes other than benzalkonium complex have been tested. For example, filters coated with a tridodecylmethyl ammonium heparin complex were tested both in vitro and ex vivo, and exhibited performance superior to uncoated filters, although the coating was found to be less effective with a polyester substrate than benzalkonium heparin complex at the same concentration. See for example FIG. 1.

The coating process may be carried out at room temperature (about 20°C). The drying temperature for the coated filter element may be between about 20°C and about 70°C.

The following examples are set forth to illustrate the invention, and are not to be construed to limit the scope of the invention.

## EXAMPLE 1

An arterial blood filter element of polyethylene terephthalate was dipped in a solution of 2-propanol containing about 2 grams of benzalkonium heparin complex per liter. The arterial filter element was immersed in the coating solution for about 1 minute. The excess solution was allowed to drain at a temperature of about 20°C, and the filter was then dried in an air-flow chamber at a temperature of about 25°C.

## EXAMPLE 2

An arterial blood filter element of polyethylene terephthalate was dipped in a solution of toluene containing about 6 grams of TDMA-heparin complex per liter. The arterial filter element was immersed in the coating solution for about 1 minute at 20°C. The excess solution was allowed to drain at ambient temperature and then dried in an oven at a temperature of 70°C.

## EXAMPLE 3

The procedure of Example 2 was repeated, but the temperature of the TDMA-heparin coating solution was kept at 55°C.

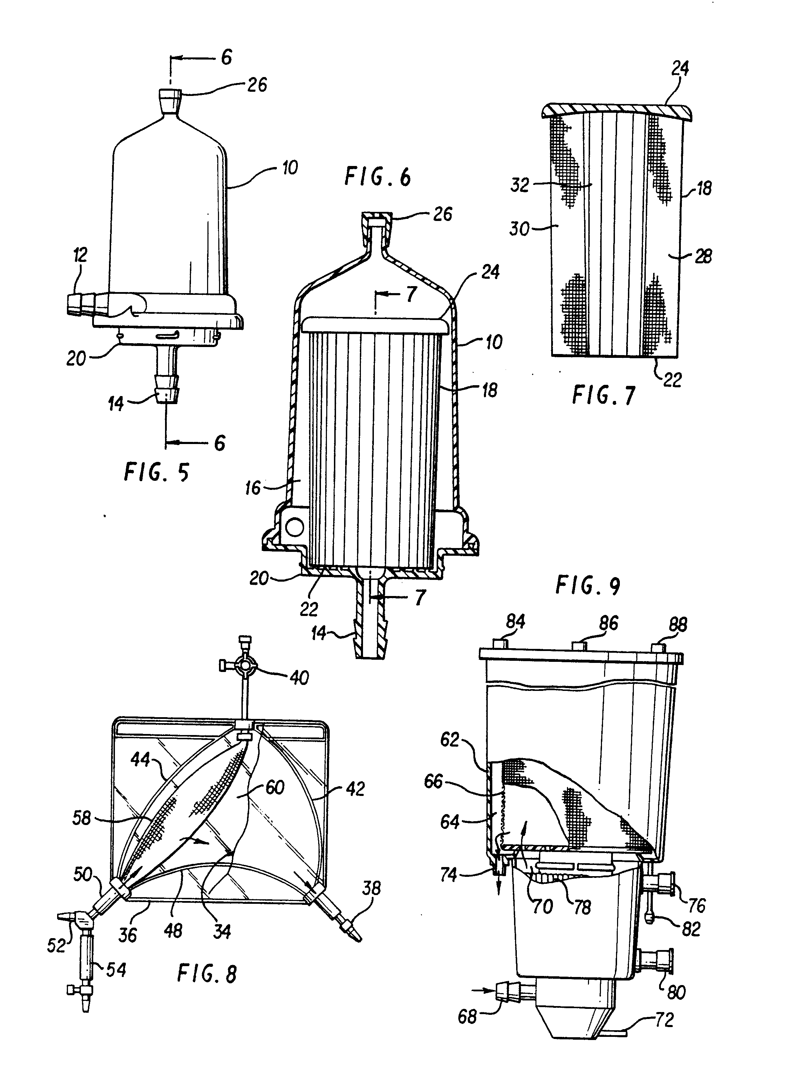

FIGURES 5, 6, and 7 illustrate an arterial blood filter with a heparin coated filter element. In FIG. 5, blood flows into the chamber defined by filter housing 10 and filter base 20 through blood inlet port 12. After being filtered, blood flows out of outlet port 14. 26 is an air vent.

FIG. 6 shows the main cavity 16 defined within housing 10. The heparin coated pleated filter element 18 is positioned within cavity 16 and is sealed at its lower pleat edge surface 22 against filter base 20. Filter cap 24 seals the top of the filter element 18. Upon entering cavity 16, blood flows through the pleats of filter 18, seen in cross-sectional view as details 28 and 30 in FIG. 7, and into the interior filter cavity 32 of the filter element. Having thus been filtered, the blood then flows downwardly through filter cavity 32 and out through outlet port 14 in FIG. 6.

Although the present invention has been described with respect to coating arterial blood filters, the invention may be applied to other blood filtration devices where improved blood compatability is desirable. FIG. 8 illustrates a cutaway plan view of another blood filtration device of the present invention, a collapsible venous blood reservoir with an integral heparin coated filter element. 34 represents a portion of a front sheet of flexible clear plastic or other material which overlays a back sheet 36 of identical shape. Front sheet 34 is heat sealed to the back sheet 36 along the perimeter of each sheet, and also along the curved reservoir cavity edges 42, 44, and 48. Inlet port 50 connects cardiotomy inlet port 52 and venous inlet port 54 with the interior of integral heparin coated filter sock 58. The integral heparin coated filter sock 58 resides within the reservoir cavity represented generally by 60, which cavity is defined by the heat sealed curved reservoir cavity edges 42, 44, and 48, and by the facing surfaces of front sheet 34 and the overlaid back sheet 36. Blood flows through inlet port 50, into integral heparin coated filter sock 58, and is then filtered into reservoir cavity 60, which can expand or contract by separation of sheets 34 and 36 to accommodate varying amounts of blood. 38 is a blood outlet port, and 40 is a venting stopcock.

FIGURE 9 is a cutaway side elevation of a blood oxygenator of the present invention. Blood oxygenator housing 62 defines a drain cavity 64 wherein resides a cylindrical integral heparin coated filter screen 66. Blood flows through inlet port 68 into inlet cavity 70, where oxygen is added to the blood through inlet oxygen inlet port 72. The oxygenated blood flows upwardly through cavity 70, into the interior of filter screen 66, and then outwardly through heparin coated filter screen 66, into drain cavity 64, where it is removed through blood outlet port 74. Heat exchanger tube 78 extends helically within inlet cavity 70 and connects heat exchanger inlet port 76 with heat exchanger outlet port 80. 82 is an arterial sampling port, and 84, 86, and 88 are gas vents.

The filter elements of the present invention can be used extracorporeally, as illustrated here, or intracorporeally. They may be used as stand-alone filters or as components in other blood handling devices which may perform significant functions in addition to the function of blood filtration. It is understood that various other modifications will be apparent to, and can readily be made by, those skilled in the art without departing from the scope and spirit of this invention. Accordingly, it is not intended that the scope of the claims appended hereto be limited to the description set forth above, but rather that the claims be construed as encompassing all the features of patentable novelty which reside in the present invention, including all features which would be art to which this invention pertains.

## Claims

1. A blood filter element suitable for use in a blood filtration device, comprising a porous filter substrate coated with a heparin-cationic surfactant complex.

2. A blood filtration device utilizing the blood filter element of claim 1.

3. The blood filtration device of claim 2, wherein said device is an integral blood filter, a blood oxygenator, or a blood reservoir.

4. A blood filter element suitable for use in a blood filter device, comprising:

a porous filter substrate;

a network of pores within said porous filter substrate suitable for the passage and filtration of blood;

a coating of heparin-cationic surfactant complex applied to the pore walls of said pores in such a manner as substantially to coat said walls without substantially occluding said pores.

5. A blood filtration device utilizing the blood filter element of claim 4.

6. The blood filter device of claim 5, wherein said device is an integral blood filter, a blood oxygenator, or a blood reservoir.

7. A blood filter element suitable for use in a blood filtration device, comprising:

a porous filter substrate;

a coating of heparin-cationic surfactant complex applied to said porous filter substrate;

a component of said heparin-cationic surfactant complex consisting of a cationic surfactant containing at least one alkyl chain of at least four carbon atoms.

8. A blood filtration device utilizing the blood filter element of claim 7.

9. The blood filter device of claim 8, wherein said device is an integral blood filter, a blood oxygenator, or a blood reservoir.

10. A blood filter element suitable for use in a blood filtration device, comprising:

a porous filter substrate;

a network of pores within said porous filter substrate suitable for the passage and filtration of blood;

a coating of heparin-cationic surfactant complex applied to the pore walls of said pores in such a manner as substantially to coat said pore walls without substantially occluding said pores;

a component of said heparin-cationic surfactant complex consisting of a cationic surfactant containing at least one alkyl chain of at least four carbon atoms.

II. A blood filtration device utilizing the blood filter element of claim I0.

I2. The blood filtration device of claim II, wherein said device is an integral blood filter, a blood oxygenator, or a blood reservoir.

I3. The blood filter element of claim I0, wherein said porous filter substrate is a foam, a screen, or a felt material.

I4. The blood filter element of claim I0, wherein said porous filter substrate is of a material selected from the group consisting of polyesters, nylons, polyolefins, acrylics, fluorinated hydrocarbons, polyurethanes, polycarbonates, or cellulose or cellulose derivatives.

I5. The blood filter element of claim I4, wherein said polyester is polyethylene terephthalate or polybutylene terephthalate.

I6. The blood filter element of claim I4, wherein said polyolefin is a polyethylene or polypropylene or a copolymer of ethylene and propylene.

I7. The blood filter element of claim I4, wherein said fluorinated hydrocarbon is polytetrafluoroethylene.

I8. The blood filter element of claim I4, wherein said cellulose derivative is cellulose acetate.

I9. The blood filter element of claim I0, wherein said pores are characterized by pore sizes ranging from about I0 microns to about I50 microns.

20. The blood filtration device of claim II, wherein said device is an arterial blood filter including a porous filter substrate having pore sizes ranging from about 20 microns to about 45 microns.

2I. The blood filtration device of claim II, wherein said device is a venous blood reservoir including a porous filter substrate having pore sizes ranging from about 80 microns to about I20 microns.

22. The blood filter element of claim I0, wherein said cationic surfactant contains at least one alkyl chain of about 8 to about I8 carbon atoms.

23. The blood filter element of claim I0, wherein said cationic surfactant is a primary, secondary, or tertiary amine, or a quaternary ammonium compound.

24. The blood filter element of claim I0, wherein said cationic surfactant is a dimethylalkylbenzyl ammonium compound, the alkyl group of said compound having a chain length of from about 8 to about I8 carbon atoms.

25. The blood filter element of claim I0, wherein said cationic surfactant is a mixture of benzalkonium cations.

26. The blood filter element of claim I0, wherein said cationic surfactant is the tridodecylmethyl ammonium cation.

27. A process for heparin coating a porous filter element for a blood filtration device, comprising:

preparing a solution of a heparin-cationic surfactant complex in a solvent, dipping said porous filter element for a blood filtration device in said solution, removing said porous filter element for a blood filtration device from said solution, and evaporating said solvent to yield a heparin coated porous filter element for a blood filtration device.

28. The process for heparin coating a porous filter element for a blood filtration device as set forth in claim 27, wherein said solvent is a lower alcohol.

29. The process for heparin coating a porous filter element for a blood filtration device as set forth in claim 27, wherein said alcohol is 2-propanol.

30. The process for heparin coating a porous filter element for a blood filtration device as set forth in claim 27, wherein said solution concentration ranges from about 0.02% to about I0% (w/v).

3I. The process for heparin coating a porous filter element for a blood filtration device as set forth in claim 27, wherein said solution concentration ranges from about 0.2 to about I% (w/v).

32. The process for heparin coating a porous filter element for a blood filtration device as set forth in claim 27, including dipping said porous filter element in said solution at about 20°C.

33. The process for heparin coating a porous filter element for a blood filtration device as set forth in claim 27, including drying said heparin coated blood filtration device at a temperature between about 40° and about 70°C.

FIG. 1

FIG. 2

FIG. 3

## INLET AND OUTLET RESIDUAL GAS (ml)
## ( MEAN AND STANDARD DEVIATION, 5 SAMPLES)

| FILTER | NON-$CO_2$ FLUSHED FILTERS | | $CO_2$ FLUSHED FILTERS | |
|---|---|---|---|---|
| | INLET(ml) | OUTLET(ml) | INLET(ml) | OUTLET(ml) |
| 25μ POLYESTER SCREEN | | | | |
| UNCOATED CONTROL FILTER | | | | |
| MEAN | 7.9 | 1.9 | 0.3 | 1.3 |
| S.D. | 2.7 | 1.2 | 0.3 | 0.5 |
| BENZALKONIUM HEPARIN COATED | | | | |
| MEAN | 0.2 | 0.0 | 0.2 | 0.2 |
| S.D. | 0.1 | 0.0 | 0.3 | 0.2 |
| 40μ POLYESTER SCREEN | | | | |
| UNCOATED CONTROL FILTER | | | | |
| MEAN | 9.6 | 2.0 | 3.4 | 1.0 |
| S.D. | 0.5 | 0.5 | 1.7 | 1.3 |
| BENZALKONIUM HEPARIN COATED | | | | |
| MEAN | 0.1 | 0.0 | 0.0 | 0.0 |
| S.D. | 0.0 | 0.0 | 0.0 | 0.0 |

## FIG. 4

0 219 053

FIG. 6

FIG. 5

FIG. 7

FIG. 9

FIG. 8